(19) Europäisches Patentamt
European Patent Office
Office européen des brevets

(11) Veröffentlichungsnummer: **0 464 759 A2**

(12) # EUROPÄISCHE PATENTANMELDUNG

(21) Anmeldenummer: **91110901.5**

(51) Int. Cl.[5]: **A61K 31/735**

(22) Anmeldetag: **01.07.91**

(30) Priorität: **03.07.90 DE 4021066**

(43) Veröffentlichungstag der Anmeldung:
**08.01.92 Patentblatt 92/02**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Diringer, Heino, Prof., Dr.**
**Havelmatensteig 21**
**W-1000 Berlin(DE)**
Erfinder: **Ehlers, Bernhardt, Dr.**
**Eisenacher Strasse 89**
**W-1000 Berlin(DE)**
Erfinder: **Schrinner, Elmar, Dr.**
**Hedwigstrasse 2**
**W-6200 Wiesbaden(DE)**
Erfinder: **Winkler, Irvin, Dr.**
**In den Eichen 40**
**W-6237 Liederbach(DE)**

(54) **Sulfatierte Polysaccharide zur Langzeitprophylaxe gegen Erkrankungen, die durch Viren oder durch unkonventionelle Viren verursacht werden.**

(57) Sulfatierte Polysaccharide und deren Derivate eignen sich zur Langzeitprophylaxe von Erkrankungen, die durch Viren oder durch unkonventionelle Viren verursacht werden.

EP 0 464 759 A2

Die vorliegende Erfindung betrifft eine Langzeitprophylaxe gegen Erkrankungen, die durch Viren oder durch unkonventionelle Viren verursacht werden. Es ist bereits bekannt, daß sulfatierte Polysaccharide und deren Derivate eine antivirale Wirksamkeit haben (DE 3601136, EP 0270317). Weiterhin ist bekannt, daß Dextransulfat und andere sulfatiertepolysaccharide eine Wirksamkeit gegen Scrapie aufweist (Ehlers, Diringer, J. Gen. Virol 65/2 (423-428), 1984). In J. Gen. Virol 65/8 (1325-1330), 1984 wird berichtet über eine zwar äußerst geringe aber dennoch signifikante Verlängerung der Inkubationszeit nach Scrapie Infektionen, wenn 10 Wochen vor der Infektion Dextransulfat verabreicht wurde.

Überraschenderweise wurde nun gefunden, daß eine Gabe von sulfatierten Polysacchariden oder deren Derivaten den Ausbruch einer Erkrankung, verursacht durch Viren oder unkonventionelle Viren verhindert, wenn die Gabe des Mittels in großem Zeitabstand vor der Infektion erfolgt. Die Gabe der genannten Mittel schützt die Tiere vor einem Angehen der Infektion für lange Zeit, im Resultat kommt die prophylaktische Gabe der Mittel einer Vakzination gleich (pseudo-Vakzination). Dies ist besonders überraschend, weil derartige Beobachtungen bisher weder bei der chemotherapeutischen Behandlung von Viruserkrankungen noch bei der Behandlung von unkonventionellen Viruserkrankungen gemacht wurden.

Das erfindungsgemäße Verfahren zur Bekämpfung von Viruserkrankungen und unkonventionellen Viruserkrankungen hat den Vorteil, daß die erfindungsgemäß verwendbaren Verbindungen im Vergleich zu anderen Antivirusmitteln wenig toxisch, teilweise sogar fast untoxisch sind. Darüber hinaus lassen sich zur erfindungsgemäßen Langzeitprophylaxe die Gaben der Polysaccharide auf mehr oder weniger regelmäßig zu gebende relativ kleine Dosen verteilen, wodurch sich unerwünschte Nebenwirkungen der Polysaccharide weitgehend oder vollständig unterdrücken lassen.

Die vorliegende Erfindung betrifft demzufolge die Verwendung von sulfatierten Polysacchariden oder deren Derivaten zur Langzeitprophylaxe von Erkrankungen, die durch Viren oder unkonvenionelle Viren verursacht werden.

Bevorzugt für die erfindungsgemäße Verwendung sind Polysaccharide, die ein Polysaccharidgerüst besitzen, das aus einheitlichen oder verschiedenen Monomereinheiten, ausgewählt aus der folgenden Gruppe von Verbindungen aufgebaut ist bzw. aus folgenden Verbindungen besteht: Xylose, Arabinose, Rhamnose, Fucose, Glucose, Mannose, Galactose, Eructose, Glucosamin, Galactosamin, Mannosamin, Glucuronsäure, Galacturonsäure, Mannuronsäure, Carrageenane, Fucoiden, Laminaran, Alginat, Lentinan, Pluran, Xylan, Dextran, Heparin, Keratansulfat, Chondroitin-4- und -6-sulfat, Dermatansulfat, Heparinsulfat, Hyaluronsäure, Teichuronsäure sowie Teilhydrolysate von Stärke, Cellulose, Glycogen, Chitin oder Pektin. Die genannten Verbindungen können sowohl synthetischer als auch natürlicher, d.h. insbesondere pflanzlicher oder mikrobieller Herkunft sein.

Weiterhin bevorzugt für die erfindungsgemäße Verwendung sind lipophil substituierte sulfatierte Polysaccharide. Unter lipophil substituierten sulfatierten Polysacchariden sind in diesem Zusammenhang insbesondere zu verstehen sulfatierte Polysaccharide, die linear oder verzweigt sein können, bestehend aus einheitlichen oder unterschiedlichen natürlichen oder synthetischen Monomeren, die pro Monomereinheit auch eine substituierte oder unsubstituierte Aminogruppe enthalten können und deren OH-Gruppen durchschnittlich zwischen 5 und 80 % substituiert sind mit einer Gruppe der Formel -X-B-Y, wobei

X eine Oxygruppe oder eine Gruppe der Formel I bedeutet

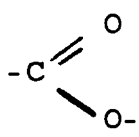

(I)

deren Kohlenstoffatom an B gebunden ist,

B einen nicht-aromatischen Kohlenwasserstoffrest mit 2 bis 30 C-Atomen bedeutet, in dessen Alkylkette bis zu 3 Methyleneinheiten durch Oxygruppen ersetzt sein können, in der bis zu drei C-C-Doppelbindungen vorliegen können und der substituiert sein kann mit bis zu drei $C_1$-$C_4$-Alkylresten und

Y - falls X eine Oxygruppe ist - Wasserstoff, COOR oder $OSO_3R^1$, sein kann, worin R ein physiologisch verträgliches ein- oder zweiwertiges Kation ist, oder einen Kohlenwasserstoffrest mit bis zu 20 C-Atomen oder einen Mono- oder Bisetherrest mit 3 bis 10 C-Atomen darstellt,
$R^1$ ein physiologisch verträgliches Kation darstellt, oder

Y - falls X ein Rest der Formel I ist - Wasserstoff oder COOR darstellt, worin R die obengenannten

Bedeutungen hat
und wobei die OH-Gruppen der obengenannten Polysaccharide zwischen 10 und 95 % substituiert sind mit einer Gruppe der Formel $OSO_3M$, wobei M ein physiologisch verträgliches Kation darstellt und zwischen 0 und 40 % der OH-Gruppen unsubstituiert sind.

Für die lipophilen Polysaccharide deren Polysaccharidgerüst bereits in der Natur in sulfatierter Form auftritt, können die sulfatierten Polysaccharide sowohl mit ihrem natürlichen Sulfatgehalt als auch nach zusätzlicher Sulfatierung verwendet werden. Die für die lipophilen sulfatierten Polysaccharide bevorzugten Polysaccharidgerüste sind die bereits oben genannten sulfatierten Polysaccharide.

Die OH-Gruppen der erfindungsgemäßen lipophilen Polysaccharide sind vorzugsweise zu 5 bis 80 %, besonders bevorzugt zu 5 bis 50 % und insbesondere zu 10 bis 40 % substituiert mit einer Gruppe der Formel -X-B-Y und weiterhin vorzugsweise zu 10 bis 95 %, besonders bevorzugt zu 45 bis 95 % und insbesondere zu 50 bis 90 % substituiert mit einer Gruppe der Formel $OSO_3M$, wobei vorzugsweise zwischen 0 und 40 %, besonders bevorzugt zwischen 0 und 20 % und insbesondere zwischen 0 und 10 % der OH-Gruppen unsubstituiert sind.

Bevorzugte Reste B weisen 2 bis 20 C-Atome auf, haben eine oder keine Doppelbindung und sind nicht oder einfach substituiert mit einer $C_1$-$C_4$-Alkylgruppe.

Sofern X eine Oxygruppe ist, ist Y vorzugsweise H, COOR, $OSO_3R^1$, wobei R ein Alkalikation, insbesondere Kalium oder Natrium oder eine verzweigte oder unverzweigte $C_1$-$C_8$-Alkylgruppe ist, $R^1$ ist vorzugsweise ein Alkalikation, insbesondere Natrium.

Falls X ein Rest der Formel I ist, bedeutet Y vorzugsweise H oder einen Rest der Formel COOR, wobei R ein Alkalikation, vorzugsweise Natrium oder Kalium, eine verzweigte oder unverzweigte $C_1$-$C_8$-Alkylgruppe oder ein Mono- oder Bisetherrest mit 6 bis 9 C-Atomen ist.

Beispielhafte Reste der Formel -X-B-Y sind Ethyloxy, Propyloxy, Isopropyloxy, Butyloxy, Hexyloxy, Octyloxy, Decyloxy, Dodecyloxy, Propionyloxy, Butyryloxy, Valeryloxy, Hexanoyloxy, Octanoyloxy, Decanoyloxy, Dodecanoyloxy, Palmitoyloxy, Stearoyloxy, 2-Methylbutyryloxy, 9-Octadecenoyloxy, Linoleyloxy, Linolenoyloxy, Cholesteryloxy, Hydroxyoxalyloxy, Hydroxymalonyloxy, Hydroxysuccinyloxy, Hydroxyglutaryloxy, Hydroxyadipoyloxy, Crotonoyloxy, Mesaconoyloxy bzw. die Ester der genannten Dicarbonsäurederivate mit unverzweigten $C_1$-$C_8$-Alkoholen oder (1,3-Diisopropoxy-propyl-2-oxycarbonyl)-propanoyloxy. Besondere Bedeutung besitzen die Dodecanoyloxy- und die Stearoyloxyreste, insbesondere an einem Xylan-Polysaccharidgerüst.

Die erfindungsgemäßen Polysaccharide in unsubstituierter oder in lipophil substituierter Form können ein mittleres Molekulargewicht aufweisen, das einen weiten Bereich überstreicht. Vorzugsweise haben die substituierten Polysaccharide ein mittleres Molekulargewicht von bis zu 100 kD, besonders bevorzugt von bis zu 40 kD, insbesondere zwischen 1 und 22 kD. Eine besondere Bedeutung haben erfindungsgemäße substituierte Polysaccharide, deren Polysaccharidgerüst aus Xylan oder Dextran besteht und die ein mittleres Molekulargewicht von 1 bis 22 kD aufweisen.

Ganz besonders bevorzugt ist Pentosanpolysulfat (polysulfatiertes ß-(1-4)-D-Xylopyranosid mit einem mittleren Molekulargewicht von 3500-5000 D).

Die Herstellung der sulfatierten Polysaccharide gehört zum Stand der Technik und ist z.B in EP 0240098, US 2715091, CH 293566 oder Carbohydr. Chem. 2, 298 (1963) beschrieben. Die Herstellung der obengenannten lipophil substituierten sulfatierten Polysaccharide erfolgt z.B. indem im Falle von Polysacchariden, die mit einer Gruppe XBY substituiert sind, wobei X eine Oxygruppe ist, die Polysaccharide unter basischer Katalyse mit einem Alkylhalogenid oder einem $\omega$-Halogencarbonsäuresalz partiell verethert werden und anschließend direkt oder nach Alkoxylierung ganz oder teilweise sulfatiert werden.

Ist X eine Gruppe der Formel I, so werden die Polysaccharide z.B. mit Carbonsäuren oder Carbonsäurederivaten partiell acyliert und die verbleibenden OH-Gruppen der Polysaccharide ganz oder teilweise sulfatiert.

Weitere Details hinsichtlich der Herstellung von lipophilen sulfatierten Polysacchariden sind in der Deutschen Patentanmeldung P 39 21 761.2 offenbart, auf die an dieser Stelle ausdrücklich Bezug genommen wird. Die in der genannten Patentanmeldung als bevorzugt beschriebenen Verbindungen sind auch für die vorliegende erfindungsgemäße Verwendung bevorzugt.

Weiterhin für die erfindungsgemäße Verwendung geeignet sind Polyelektrolytkomplexe aus einer Polysäure und einer Polybase. Bevorzugte Polysäuren sind die bereits oben beschriebenen sulfatierten Polysaccharide in substituierter oder unsubstituierter Form. Besonders bevorzugte sulfatierte Polysaccharide haben einen Sulfatierungsgrad von mehr als 10 %, besonders bevorzugt 90 bis 100 %.

Das mittlere Molekulargewicht der unsubstituierten und substituierten Polysaccharide für die Polyelektrolytkomplexe sollte zwischen 500 und 80000 D, bevorzugt zwischen 1000 und 40000 D, insbesondere zwischen 4000 und 15000 D liegen.

Die erfindungsgemäßen Polyelektrolytkomplexe können mit Hilfe unterschiedlicher Polybasen gebildet werden. U.a. können Proteine als Polybasen eingesetzt werden, wie z.B. Hämoglobin. Weiterhin sind z.B. Polylysin, Lysinalkylester, Chitosan, Poly-(2,N,N-Dimethyl-aminoethyl)D,L-aspartamid, Aminosäuren, Polyaminosäuren, aminierte Oligo- und Polysaccharide (z.B. aminierte Dextrane, Poly-[α,β-(spermidinyl)-D,L-aspartemid], Gelatine und Derivate (z.B. Polygeline®, Behringwerke AG, Marburg), quarternäre Ammoniumverbindungen (z.B. Luviquat®, BASF AG, Ludwigshafen oder Micrapol A15®, Miranal Chem. Co., Inc. South Brunswick, New Yersey, $C_{12}$-Alkylsternamin, α,ω-Bis-(N-propylaminopolyethylenglycol sowie aminierte Polyethylenglycolderivate, geeignet. Weiterhin können als Polybasen kovalente Verbindungen aus OH-Gruppenhaltigen Polymeren (z.B. Dextran) und Stickstoffkronenethern sowie deren Metallkomplexe eingesetzt werden.

Weiterhin ist es möglich, die erfindungsgemäßen Polyelektrolytkomplexe durch Variation des Substituenten der Polysäuren und Polybasen in Bezug auf ihre Resorptionsfähigkeit zu optimieren. So ist es z.B. möglich, die Polysäuren mit unterschiedlichen Substituenten, wie oben bereits erläutert, auszustatten. Die Polybasen können ebenfalls durch Substitutionsreaktionen verändert werden. Besonders geeignete Substituenten für die Polybasen sind hydrophobisierender Natur, wie z.B. Alkylketten, insbesondere verzweigte oder unverzweigte Alkylketten mit 10-20 C-Atomen, wie z.B. Lysinoctadecylester oder Cetyltrimethylammonium-bromid. Andere geeignete Substituenten sind z.B. Steroidreste, insbesondere Cholesterol über die Alkoholgruppe gebunden bzw. der Cholesterylester der Bernsteinsäure oder analoge Steroide.

Darüber hinaus ist es möglich, maßgeschneiderte Polyelektrolytkomplexe, hergestellt durch die Umsetzung einer Polysäure oder einer Mischung von Polysäuren mit einer Polybase oder einer Mischung von Polybasen einzusetzten.

Die Herstellung der Polyelektrolytkomplexe, erfolgt z.B. indem entweder a) die Polysäure oder b) die Polybase vorzugsweise in wäßriger Lösung vorgelegt wird und a) die Polybase oder b) die Polysäure bei geeigneter Temperatur und bei geeignetem pH-Wert vorzugsweise in wäßriger Lösung zugetropft wird.

Weitere Details hinsichtlich der Polyelektrolytkomplexe sind in der deutschen Patentanmeldung P 39 37 283.9, auf die an dieser Stelle ausdrücklich Bezug genommen wird, beschrieben. Die in der genannten Patentanmeldung als bevorzugt herausgestellten Polyelektrolytkomplexe sind auch für die vorliegende erfindungsgemäße Verwendung bevorzugt.

Die erfindungsgemäß verwendbaren Verbindungen dienen zur Prophylaxe von Erkrankungen die durch Viren oder unkonventionelle Viren hervorgerufen werden. Besondere Bedeutung besitzen sie für die Langzeitprophylaxe von Erkrankungen die durch Herpesviren oder durch Retroviren wie z.B. HTLV I und II, HIV, BLV, FLV, FeLV, FIV, Visna-Maedi-Viren, Goat-Arthritis-Viren, humane Spuma-Viren oder durch unkonventionelle Viren hervorgerufen werden.

Ganz besondere Bedeutung besitzen sie für die Bekämpfung von Retroviruserkrankungen und Erkrankungen im Zusammenhang mit unkonventionellen Viren, insbesondere gegen Scrapie, die Bovine Spongioforme Encephalopathy (BSE-Virus) und das Jacob-Creutzfeldt-Syndrom.

Langzeitprophylaxe bedeutet in dem vorliegenden Zusammenhang, daß die betreffenden Verbindungen mindestens einen, vorzugsweise drei, insbesondere fünf Tage vor einer potentiellen Infektion gegeben werden.

Vorzuziehen ist es jedoch, die betreffenden Mittel weit vor einer potentiellen Infektion nach Gabe einer oder mehrerer Initialdosen in regelmäßigen Abständen in relativ geringer, nebenwirkungsarmer Dosis dem infektionsgefährdeten Organismen zu verabreichen. Auf diese Weise werden die betreffenden Organismen optimal gegen das Ausgehen einer Infektion geschützt, ohne daß unerwünschte Nebenwirkungen auftreten. Die Gabe der Verbindungen kann vorzugweise in einem zeitlichen Abstand von 90 Tagen, besonders bevorzugt von 80 Tagen, inbesondere von 60 Tagen erfolgen. Die Dosierung bei den einzelenen Gaben liegt mengenmäßig unter der Dosis, die zur Bekämpfung einer angegangenen Infektion nötig wäre. Die bevorzugte Initialdosis liegt bei 0,1-10 besonders bevorzugt bei 3-9, insbesondere bei ca. 4 mg/kg Körpergewicht eines Säugetierorganismus. Die bevorzugte Erhaltungsdosis, die nach einem wie oben erläuterten Zeitabstand zu geben ist, liegt bei 0,1-5, besonders bevorzugt bei 0,5-2, insbesondere bei ca. 1,5 mg/kg Körpergewicht. Die beschriebenen Dosierungen gelten für die Verabreichung durch Injektion; bei anderer Verabreichungsart kann es nötig sein, die Dosen zu erhöhen, z.B. um das 5-10 fache.

Die erfindungsgemäß verwendbaren Verbindungen können z.B. oral, intravenös, intramuskulär, intraperitoneal, subkutan oder rektal verabreicht werden.

Weiterhin betrifft die vorliegende Erfindung Arzneimittel zur Langzeitprophylaxe von Erkrankungen, die durch Viren oder unkonventionelle Viren verursacht werden, die durch einen Gehalt an erfindungsgemäß verwendbaren Verbindungen gekennzeichnet sind. Die Herstellung der erfindungsgemäßen Arzneimittel erfolgt, indem mindestens ein substitueirtes Polysaccharid oder eines seiner Derivate gegebenenfalls mit

weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und andere üblicher Hilfsstoffe.

Zum Beispiel können für orale Darreichungsformen Hilfsstoffe wie Stärken, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Daüber hinaus kann es vorteilhaft sein, der Darreichungform, ein Retardierungsmittel zuzusetzen, gegebenenfalls in Form von permeablen Membranen, z.B solcher auf Cellulose- oder Polystyrolharzbasis oder Ionenaustauscher. Weiterhin kann es sinnvoll sein, die erfindungegemäß verwendbaren Verbindugnen in Form eines Implantates zu verabreichen.

Durch die nachfolgenden Ausführungsbeispiele soll die vorliegende Erfindung näher erläutert werden.

Die vorliegende Erfindung wird durch die nachfolgenden Ausführungsbeispiele sowie durch die Patentansprüche erläutert.

**Beispiel 1:**

Auswirkung der prophylaktischen Gabe von Pentosanpolysulfat (MW ca. 5000 Dalton) auf die Splenomegalie nach Infektion mit Friend-Leukämievirus (FLV).

3 Gruppen von je 10 weibliche NMRI-Mäusen, Körpergewicht 18-20 g, wurden jeweils 50, 40 und 30 Tage vor Infektion durch intra-peritoneale Injetion von Pentosanpolysulfat in physiologischer Kochsalzlösung behandelt:

Gruppe 1: 0 mg/kg (Kontrolle)
Gruppe 2: 50 mg/kg
Gruppe 3: 250 mg/kg
Gruppe 4: 500 mg/kg

Die Infektion erfolgte durch intravenöse Injektion von 0,2 ml eines 1:10000 verdünnten Serums aus FLV infizierten NMRI-Mäusen. 14 Tage nach Infektion wurden die Milzen der Versuchstiere gewogen. Das Milzgewicht diente als Maßeinheit für die Vermehrung des FLV in den Versuchstieren (Chirgos M.A., Luber, E., March, R., Pettigrew, H. (1965) Antiviral chemotherapeutic assay with Friend Leukemia virus in mice. Cancer Chemother. Rep. 45, 29-33). Das Ergebnis ist in folgender Tabelle (1) zusammengefaßt:

| Gruppe | Dosierung mg/kg | Milzgewicht ± S.D. | Überlebende |
|---|---|---|---|
| 1 | 0 | 2,54 ± 0,37 | 10 |
| 2 | 50 | 1,77 ± 0,61 | 8 |
| 3 | 250 | 1,67 ± 0,89 | 4 |
| 4 | 500 | 1,56 ± 0,53 | 5 |

Während das Milzgewicht von nicht infizierten Mäusen ca. 0,1 g beträgt, entwicklen FLV infizierte Mäuse innerhalb von 2 Wochen eine Splenomegalie mit Milzgewichten von über 2 g (Gruppe 1).

Die Vorbehandlung mit Pentosanpolysulfat führte dosisabhängig zu einer statistisch signifikanten ($p \leqq 0,05$) Reduktion des Milzgewichts gegenüber der unbehandelten Kontrolle (Gruppe 1). Es kann daraus geschlossen werden, daß eine Vorbehandlung der Mäuse mit Pentosanpolysulfat eine nachfolgende Infektion mit FLV beeinflußt hat. Die während des Versuches beobachteten Todesfälle bei den Versuchstieren traten als Folge der antikoagulatorischen Wirkung von Pentosanpolysulfat bei hoher Dosierung bereits vor der Infektion auf.

**Beispiel 2**

Prophylaktische Wirkung von Pentosanpolysulfat (MW: 5000 Dalton) auf Scrapie-Infektionen bei Mäusen.

In 2 unabhängigen Experimenten wurden Gruppen von je 6-8 Mäusen durch intraperitoneale Gabe von jeweils 10 mg/Maus Pentosanpolysulfat an folgenden Tagen vor der Infektion mit dem Scrapie-Agens vorbehandelt:

| Experiment 1 | Experiment 2 |
|---|---|
| - 84<br>- 77<br>- 71 | - 70<br>- 60<br>- 50 |

Entsprechende Kontrollgruppen wurden zu den angegebenen Zeitpunkten mit phosphatgepufferter Kochsalzlösung behandelt.
Die Infektion erfolgte durch intraperitoneale Injektion von 0,1 ml Homogenat aus Scrapie infizierten Mäusen in einer Verünndung von 1:100 bis 1:1 Mio. Die Beobachtungszeit betrug 384 (Experiment 1) bzw. 307 Tage (Experiment 2), während der die Versuchstiere zweimal wöchtenlich, bei Beginn der Symptome täglich, auf ihren Gesundheitszustand und mögliche Anzeichen einer Scrapie-Infektion hin überprüft wurden. Die Überlebensdauer und die Anzahl der überlebenden Tiere diente als Maß für die Wirkung der Prophylaxe mit Pentosanpolysulfat.
Das Versuchsergebnis ist in folgender Tabelle (2) zusammengefaßt:

| | Inkubationszeit (Tage) und Überlebensrate | | | |
|---|---|---|---|---|
| Infektions- | Experiment 1 | | Experiment 2 | |
| dosis | Kontrolle | behandelt | Kontrolle | behandelt |
| 10-2 | 155 ± 1 | 239 ± 9 | 167 ± 2 | 220 ± 6 |
| | 0 / 7 | 0 / 6 | 0 / 6 | 1 / 7 |
| 10-3 | 160 ± 2 | 248 ± 9 | 174 ± 3 | 268 ± 15 * |
| | 0 / 7 | 1 / 6 | 0 / 7 | 5 / 8 |
| 10-4 | 184 ± 5 | - | 187 ± 3 | 267 ± |
| | 0 / 7 | 6 / 6 | 1 / 7 | 7 / 8 |
| 10-5 | 187 ± 4 | - | 186 ± 2 | - |
| | 2 / 6 | 6 / 6 | 4 / 7 | 7 / 7 |
| 10-6 | | | - | - |
| | | | 7 / 7 | 7 / 7 |

*) n = 3, ein Tier vorzeitig mit anderer Symptomatik verstorben

Es konnte gezeigt werden, daß in beiden Experimenten die Vorbehandlung mit Pentosanpolysulfat die Inkubationszeit gegenüber der jeweiligen Kontrolle verlängert oder, bei geringerer Infektionsdosis den Ausbruch der Scrapie-Erkankung verhinderte.
Weitere Analysen der Tiere aus Experiment 2 zeigten, daß die im Versuchszeitraum nicht erkrankten Tiere kein Amyloid-Protein aufwiesen, während bei allen Kontrollen und den behandelten, erkrankten Mäusen Amyloid nachweisbar war. Die Anhäufung von Amyloid-Protein im Gehirn gilt als wichtiges Kriterium für das Vorliegen einer Scrapie-Erkrankung.

**Patentansprüche**

1. Verwendung von sulfatierten Polysacchariden oder deren Derivaten zur Langzeitprophylaxe von Erkrankungen, die durch Viren oder unkonventionelle Viren verursacht werden.

2. Verwendung gemäß Anspruch 1, dadurch gekennzeichnet, daß das sulfatierte Polysaccharid aus einheitlichen oder verschiedenen Monomereinheiten ausgewählt aus der folgenden Gruppe von Verbindungen aufgebaut ist oder besteht: Xylose, Arabinose, Rhamnose, Fucose, Glucose, Mannose, Galactose, Fructose, Glucosamin, Galactosamin, Mannosamin, Glucuronsäure, Galacturonsäure, Mannuronsäure, Carrageenane, Fucoiden, Laminaran, Alginat, Lentinan, Pluran, Xylan, Dextran, Heparin, Keratansulfat, Chondroitin-4- und -6-sulfat, Dermatansulfat, Heparinsulfat, Hyaluronsäure, Teichuronsäure sowie Teilhydrolysate von Stärke, Cellulose, Glycogen, Chitin oder Pektin.

**3.** Verwendung gemäß Anspruch 1, dadurch gekennzeichnet, daß das Derivat des sulfatierten Polysaccharids ein liphophil sulfatiertes Polysaccharid ist.

**4.** Verwendung gemäß Anspruch 1, dadurch gekennzeichnet, daß das Derivat des sulfatierten Polysaccharids ein Polyelektrolytkomplex ist.

**5.** Verwendung gemäß einem oder mehreren der Ansprüche 1-4 dadurch gekennzeichnet, daß das Virus ein Retrovirus, insbesondere HIV, ist.

**6.** Verwendung gemäß einem oder mehreren der Ansprüche 1-5, dadurch gekennzeichnet, daß das unkonventionelle Virus der Erreger von Scrapie, des Jacob-Creutzfeldt-Syndroms oder der spongiformen Enzephalopathie ist.

**7.** Verwendung von sulfatierten Polysacchariden oder deren lipophilen Derivaten zur Herstellung eines Langzeitprophylaktikums gegen Krankheiten, die durch Viren oder durch unkonventionelle Viren verursacht werden.

**8.** Arzneimittel, enthaltend ein sulfatiertes Polysaccharid oder eines seiner Derviate zur Langzeitprophylaxe von Erkrankungen, die durch Viren oder durch unkonventionelle Viren hervorgerufen werden.

**9.** Verfahren zur Langzeitprophylaxe von Erkrankungen, die durch Viren oder unkonventionelle Viren verursacht werden, dadurch gekennzeichnet, daß ein sulfatiertes Polysaccharid oder eines seiner Derivate verabreicht wird.